Europäisches Patentamt

European Patent Office

Office européen des brevets

(19)

(11) Veröffentlichungsnummer: **0 298 148 B1**

(12) **EUROPÄISCHE PATENTSCHRIFT**

(45) Veröffentlichungstag der Patentschrift: **18.09.91**

(51) Int. Cl.⁵: **C07H 15/256**, C07G 3/00

(21) Anmeldenummer: **87110015.2**

(22) Anmeldetag: **10.07.87**

(54) Verfahren zur Gewinnung von Beta-aescinreichen Extrakten.

(43) Veröffentlichungstag der Anmeldung:
**11.01.89 Patentblatt 89/02**

(45) Bekanntmachung des Hinweises auf die
Patenterteilung:
**18.09.91 Patentblatt 91/38**

(84) Benannte Vertragsstaaten:
**AT CH DE FR GB IT LI**

(56) Entgegenhaltungen:
DE-A- 2 915 160
FR-A- 1 470 308
GB-A- 1 539 127
US-A- 3 450 691

**ARCHIV DER PHARMAZIE, Band 304, 1971,
Seiten 347-350, Weinheim; H. FRIEDRICH et
al.: "Zur Variabilität des "Aescin"-Gehaltes
in den Samen von Aesculus Arten"**

(73) Patentinhaber: **Herbe Wirkstoff GmbH
Prinz-Handjery-Strasse 22
W-1000 Berlin 37(DE)**

(72) Erfinder: **Horvath, Erich, Dr.
Prinz-Handjery-Strasse 22
W-1000 Berlin 37(DE)**

(74) Vertreter: **Freiherr von Pechmann, Eckehart et
al
Wuesthoff & Wuesthoff Patent- und Rechtsanwälte Schweigerstrasse 2
W-8000 München 90(DE)**

## Beschreibung

Die therapeutische Wirkung zahlreicher Venenpräparate wird nach ihrem Aescingehalt bewertet. Diese in den Früchten oder Samen der Roßkastanie (Aesculus hippocastanum L.) enthaltene Wirkstoff stellt ein komplexes Gemisch nahverwandter Triterpenglykoside dar, die in isomeren Formen existieren können (J. Wagner et alk, Arzneim.-Forsch. 20, 205 (1970). Wie man heute weiß, stellt eines dieser Isomere die genuine Form dar, die unter der Bezeichnung ß-Aescin in die Literatur eingegangen ist (The Merck Index, No. 3640, Tenth Edition (1983). Da von den isomeren Aescinformen das ß-Aescin die stärkste pharmakologische Aktivität aufweist (H. Hampel et al., Arzneim.-Forsch. 20, 209 (1970)), hat man dieses Isomer bei Aescinpräparaten bevorzugt eingesetzt.

Aufgrund der erheblichen therapeutischen Bedeutung dieser Präparate hat es nicht an Versuchen gefehlt, die seit langem bekannten Verfahren zur Gewinnung dieses Wirkstoffs zu verbessern. Hierbei werden die zerkleinerten frischen Samen der Raßkastanie zunächst mit wässrigen Alkoholen extrahiert und anschließend aus dem Extrakt das Aescin gewonnen.

Von entscheidender Bedeutung ist hierbei der Aescingehalt im Samenmaterial, vor allem der Gehalt an ß-Aescin. Nach Untersuchungen von H. Friedrich und E. Zeruhn (Arch. Pharmazie 304, 347 (1971)) unterliegt der Aescingehalt von Samen verschiedener Bäume der Art Aesculus hippocastanum L. erheblichen Schwankungen. Wie nach erschöpfender Extraktion an gefriergetrocknetem pulverisiertem Samenmaterial festgestellt werden konnte, liegt er bei den einzelnen Individuen zwischen 1,8% und 7,99%, im Durchschnitt bei 3,9%. Ähnliche Ergebnisse werden auch in der DE-PS 27 33 204 "Verfahren zur Isolierung von Aescin aus Früchten der Roßkastanie" mitgeteilt. Hier verweist man darauf, daß die getrockneten "Früchte" (gemeint sind Samen) 3,5% bis 5,5% Gesamtaescin enthalten, wobei der Anteil der isomeren Form alpha-Aescin von weniger als 20% bei frischen Kastanien auf 50% bei älteren Früchten ansteigen kann. Das bedeutet, daß der Anteil der isomeren Form ß-Aescin von mehr als 80% bei frischen Kastanien auf 50% bei länger gelagerten Früchten sinken kann.

In einer ausführlichen Studie zur industriellen Produktion von aescinreichen Roßkastanienextrakten berichten U. Bogs und D. Bremer (Pharmazie 26, 410 (1971)), daß man aus luftgetrockneten, geschroteten Roßkastaniensamen durch Perkolation mit 70%igem Ethanol einen Extrakt gewinnen kann, der, auf die eingesetzte Droge bezogen, 1,5% Aescin enthält.

Für die Isolierung des Aescins aus den Roßkastaniensamenextrakten sind verschiedene Verfahren entwickelt worden (Literaturübersicht in: J. Wagner und J. Bosse, Hoppe-Seyler's Ztschr. physiol. Chem. 320, 27 (1960)). Im speziellen Fall der Gewinnung von ß-Aescin nützt man die geringe Wasserlöslichkeit der freien Säureform von ß-Aescin aus. Dies gelingt bereits durch Einstellen des an sich fast neutralen Roßkastaniensamenextraktes auf pH-Werte 3, z.B. durch Zugabe entsprechend starker Säuren `der durch Behandeln mit einem stark sauren Ionenaustauscher. In der DDR-PS 11 178 "Verfahren zur Gewinnung von kristallisiertem Saponin (Aescin) aus den Samen der Roßkastanie" wurde durch Ansäurern eines methanolisch-wässrigen Roßkastaniensamenextraktes kristallisiertes Aescin in einer Ausbeute von ca. 2,5% (bezogen auf die eingesetzte Droge) erhalten. Nach W. Winkler und P. Patt (Naturwissenschaften 47, 83 (1960)) fällt kristallines Aescin nach Behandeln wässrig-methanolischer Roßkastaniensamenextrakte mit Ionenaustauscher in einer Ausbeute von ca. 2% (bezogen auf die eingesetzte Droge) an. Später berichten G. Kamphius, P. Patt und W. Winkler (Österreichische Apoth.-Ztg. 24, 535 (1970)) von einer durchschnittlichen Ausbeute von 1,5% kristallisiertem Aescin.

Im allgemeinen erfolgt die technische Aescinproduktion unter Anwendung folgender Verfahrensschritte:

1. Luft-/Wärmetrocknung von vorzerkleinerten Kastanien; 2. Weitere Zerkleinerung der Kastanien und Extraktion mit 40 - 70%igem Alkohol (Methanol oder Ethanol); 3. Isolierung von Gesamtaescin (Säurefällung, Ionenaustauscher oder Verteilung); 4. Abtrennung des alpha-Aescin-Anteils und Gewinnung der ß-Aescin-Handelsspezifikation.

Obwohl bereits bekannt ist, daß Roßkastaniensamen durchschnittlich 4% Aescin enthalten, und im frischen Zustand der Droge mehr als 80% davon in der ß-Form vorliegt, ist bisher kein Produktionsverfahren beschrieben worden, das eine weitgehende Gewinnung dieses genuinen, also ursprünglich in frischen Roßkastaniensamen vorhandenen ß-Aescins ermöglicht.

Überraschenderweise wurde nunmehr festgestellt, daß sich das zerkleinerte Kastanienmaterial mit Wasser als Lösungsmittel erschöpfend extrahieren läßt und das anschließend aus dem Extrakt isolierte Aescin - ohne Abtrennung eines alpha-Aescin-Anteils - der ß-Aescin-Handelsspezifikation entspricht, wenn zur Extraktion frische gefrorene Kastanien verwendet werden. Die frischen Kastanien werden nach der Ernte sogleich tiefgefroren und lassen sich in dieser form gut lagern und auch transportieren. Nach dem Auftauen werden die Samen zerkleinert, z.B. zur Vergrößerung der Extraktionsoberfläche in dünne Scheiben geschnitten, und dann mit Wasser unter Rühren oder Umlauf des Extraktionswassers extrahiert. Die Dauer dieser Extraktionsstufe, bei der jetzt das Aescin aus dem gefrorenen und wieder aufgetauten zerkleinerten Kastaniengewebsmaterial durch das Wasser praktisch vollständig herausgelöst wird, hängt u.a. von der Kastaniensorte, dem Zerkleinerungsgrad und der Extraktionstemperatur ab, und kann 3 - 50 Stunden betragen. Durch Probeextraktion des jeweiligen Materials läßt sich die optimale Zeitdauer für diese Extraktionsstufe leicht ermitteln. Aus dem erhaltenen filtrierten oder zentrifugierten Extrakt wird unverzüglich durch Ansäuern das gelöste Aescin ausgefällt und dieses in üblicher Weise durch Umkristallisieren usw. zum reinen Wirkstoff aufgearbeitet.

Obwohl das Tiefgefrieren und Konservieren von pflanzlichem Material seit längerer Zeit bekannt ist, hat man dies bisher bei den Kastaniensamen nicht angewendet. Auch war nicht vorherzusehen, daß durch das Tiefgefrieren nach dem Auftauen es möglich sein wird, das Aescin anstelle mit Alkohol bzw. mit wässrigem hochprozentigen Alkohol oder anderen mit Wasser mischbaren polaren organischen Lösungsmitteln jetzt allein mit Wasser als Extraktionsmittel aus dem Samenmaterial herauszulösen, und zwar mit besserer Ausbeute als früher mit den organischen Extraktionsmitteln.

Das erfindungsgemäße Verfahrensprinzip "Tieffrieren der Kastanien und Extraktion mit Wasser" führt gegenüber dem Stand der Technik in mehrfacher Hinsicht zu technischen und wirtschaftlichen Fortschritten:

1.) Das Trocknen der Kastanien, das mit einer teilweisen Isomerisierung von ß-Aescin zu kommerziell wertlosem alpha-Aescin verbunden ist, entfällt.

2.) Die Tieffrierlagerung der frischen Kastanien ist eine Lagertechnik, bei der die Isomerisierung des Aescins während der Lagerung verhindert wird, so daß die Aufarbeitung der geernteten Kastanien jetzt je nach Bedarf erfolgen kann.

3.) Das Hantieren von großen Mengen an Alkoholgemischen entfällt, so daß Rückgewinnung des Alkohols, gesundheitliche Schutzvorkehrungen und Schutzeinrichtungen nicht erforderlich sind und lediglich bei der Endreinigung von Aescin, wo sich die zu verarbeitenden Mengen auf etwa ein Hundertstel reduziert haben, Alkohol noch zum Einsatz kommt.

4.) Wasser ist das kostengünstigste und unschädlichste Lösungsmittel für die Extraktion.

5.) Die Stufe der Abtrennung von alpha-Aescin entfällt.

6.) Die Ausbeute an ß-Aescin ist wesentlich gesteigert.

Im technischen Maßstab bringt die Extraktion des aufgetauten tiefgefrorenen Kastanienmaterials mit Wasser die Gefahr einer möglichen Entwicklung von Mikroorganismen mit sich, zumal die Kastanien auch niedermolekulare Zucker und die verschiedensten Nährstoffe für eine Gärung enthalten, die besonders bei Extraktionstemperaturen von 20 - 40°C leicht eintreten können.

Durch eine solche Gärung könnte es zu einer unkontrollierten Absenkung des ansonsten nahezu neutralen (5,5 - 6,5) pH-Werts kommen, verbunden mit unübersichtlichen Abbaureaktionen und Isomeriergefahr für das genuine Aescin. Daher empfiehlt es sich, dem Extraktionswasser eine geringe Menge eines das Bakterien- oder Hefewachstum verhindernden Mittels zuzusetzen oder die nahezu aufgetauten, ganzen Kastanien äußerlich mit einem handelsüblichen Desinfektionsmittel zu behandeln, um die Grundkeimbelastung zu reduzieren, worauf anschließend die Kastanien schnell in Scheiben geschnitten und der erschöpfenden Extraktion mit dem Wasser unterworfen werden. Wenn längere Extraktionszeiten vorgesehen sind, soll aber das Extraktionswasser das Desinfektionsmittel enthalten. Als ein solches Gärung verhinderndes Mittel können dem Extraktionswasser u.a. geringe Mengen Phenol oder Nipagin sowie auch niedere Alkohole, wie Methanol, Ethanol oder Isopropanol in einer Menge, die eine Entwicklung von Bakterien oder Hefe während der Extraktionsdauer sicher verhindert, zugesetzt werden. Bei den niederen Alkoholen ist dies bekanntlich bei einer Konzentration von 14 - 20 Vol.-% sicher der Fall.

Beispiel 1

10.000 kg frische, ganze Kastanien werden tiefgefroren und dann gelagert. Die angetauten Kastanien, die sich üblicherweise in Jutesäcken befinden, werden partienweise ca. 30 Minuten in eine 1%ige wässrige Lösung des handelsüblichen Desinfektionsmittels "Perform Neu" der Firma Schülke & Mary GmbH - enthaltend Kaliumperoxymonosulfat, Natriumbenzoat und Weinsäure - getaucht. Nach Abtropfen werden die Kastanien mit einer Schneidemaschine zu dünnen Scheiben von ca. 3 mm Stärke geschnitten und in 40.000 l vorgelegtes Wasser eingetragen. Nach 5 Stunden Extraktion unter Umpumpen wird die Tinktur abgezogen, Mittels Zentrifugieren von Schwebeteilen befreit und unmittelbar danach bei ca. 45°C und Zugabe von Schwefelsäure bis zum pH-Wert von 2,5 - 2,8 das Aescin gefällt. Nach Absetzen über Nacht wird der klare Überstand entfernt, die verbleibende Aescinsuspension filtriert und und der Filterkuchen getrocknet. Der Filterkuchen wird unter Rühren in Methanol gelöst, die Lösung zur Entfärbung z.B. mit Aktivkohle behandelt und das Lösungsmittel bis zur Trockene entfernt.

Ausbeute:     178 kg Aescin, d.s. 1,78% bezogen auf frische Kastanien und ca.

3,6% bezogen auf Kastanien trokkensubstanz

Qualität: Entspricht der ß-Aescin-Handelsspezifikation, d.h. das erhaltene Aescin besteht zu 88,1 % aus ß-Aescin.

Beispiel 2

10.000 kg frische, ganze Kastanien werden tiefgefroren gelagert. Nach dem Auftauen werden sie grob zerkleinert (z.B. Walzenstuhl, Walzenabstand 10 mm) und in 8.000 l Wasser eingetragen, dem als Desinfektionsmittel Methanol zugesetzt wurde, so daß unter Berücksichtigung des Wassergehalts der Kastanien sich eine Methanolkonzentration von 15% (V/V) ergibt. Unter Umpumpen wird 36 Stunden lang extrahiert, die Tinktur abgezogen und filtriert. Der vorgenannte Extraktionsprozeß wird dann noch zweimal mit jeweils 8.000 l 15% Methanol enthaltendem Wasser wiederholt.

Aus den vereinigten Tinkturen wird bei ca. 40°C und pH 2,5 - 2,8 (Zugabe von Schwefelsäure) Aescin gefällt. Die über Nacht abgesetzte Fällsuspension wird filtriert. Der feuchte Filterkuchen wird unter Rühren und Zugabe von Isopropanol gelöst, mit z.B. Aktivkohle zur Entfärbung behandelt, filtriert und das Lösungsmittel bis zur Trockene eingedampft.

Ausbeute: 196 kg Aescin, d.s. 1,96% bezogen auf frische Kastanien und ca. 3,9% bezogen auf kastanientrokkensubstanz

Qualität: Entspricht der ß-Aescin-Handelsspezifikation und besteht zu 88,6 % aus ß-Aescin.

## Patentansprüche

1. Verfahren zur Gewinnung von ß-aescinreichen Extrakten durch Extrahieren zerkleinerter Kastaniensamen und Ausfällen des Aescins durch Ansäuern des Extrakts, dadurch **gekennzeichnet,** daß man frische tiefgefrorene Roßkastaniensamen verwendet, die nach dem Auftauen und Zerkleinern dann mit Wasser extrahiert werden.

2. Verfahren nach Anspruch 1, dadurch **gekennzeichnet,** daß dem Extraktionsmittel Wasser zur Vermeidung von mikrobiellem Wachstum bei der Extraktion Desinfektionsmittel zugesetzt werden.

3. Verfahren nach Anspruch 2, dadurch **gekennzeichnet,** daß als Desinfektionsmittel niedere Alkohole, insbesondere Methanol oder Ethanol, in einer Menge bis zu 20 Vol.-% verwendet werden.

## Claims

1. A method of producing extracts rich in ß-escin by extracting crushed chestnut seeds and precipitating the escin by acidifying the extract, **characterized** in that fresh, deep-frozen horse chestnut seeds are used which are extracted with water after having been thawed and crushed.

2. The method as claimed in claim 1, **characterized** in that disinfectants are added to the extracting agent water to prevent microbial growth during the extraction.

3. The method as claimed in claim 1, **characterized** in that low alcohols, specifically methanol or ethanol, in a quantity of up to 20 percent by volume are used as disinfectants.

## Revendications

1. Procédé pour la fabrication d'extraits riches en ß-aescin par extraction de graines de châtaignes broyées et précipitation de l'aescin par acidification de l'extrait, **caractérisé** en ce que l'on use des graines de châtaignes fraîches, surgelées qui sont extraites par l'eau après avoir été décongelées et broyées.

2. Procédé selon la revendication 1, **caractérisé** en ce que l'eau, l'agent d'extraction, est additionnée d'un désinfectant pour éviter la croissance microbienne pendant l'extraction.

3. Procédé selon la revendication 2, **caractérisé** par l'usage d'alcools inférieurs, notamment du méthanol ou de l'éthanol, en quantité jusqu' à 20 pourcent par volume, comme désinfectant.